# EUROPEAN PATENT APPLICATION

(11) **EP 4 809 832 A1**
(43) Date of publication of application: **23.09.2026**
(21) Application number: 25867445.6
(22) Date of filing: 19.03.2025
(51) Int. Cl.: A23L 33/135, A23L 33/125, A61K 35/745, A61K 31/702, A61P 1/00

(54) **COMPOSITION COMPRISING HUMAN MILK OLIGOSACCHARIDE AND PROBIOTIC, AND USE OF COMPOSITION IN IMPROVING INTESTINAL DEVELOPMENT**

(30) Priority: 20.09.2024 CN 202411312231
(71) Applicant: Ausnutria Dairy (China) Co., Ltd., Changsha, Hunan 410127 (CN); Bioflag Co., Ltd., Huai'an, Jiangsu 223010 (CN)
(72) Inventor: WA, Yunchao, Changsha, Hunan 410127 (CN); PENG, Xiaoyu, Changsha, Hunan 410127 (CN); WANG, Jiaqi, Changsha, Hunan 410127 (CN); LIU, Min, Changsha, Hunan 410127 (CN); ZHAO, Xia, Changsha, Hunan 410127 (CN); XIE, Yu, Changsha, Hunan 410127 (CN); PAN, Lina, Changsha, Hunan 410127 (CN); LI, Wei, Changsha, Hunan 410127 (CN); GU, Ruixia, Changsha, Hunan 410127 (CN); YANG, Zhenquan, Changsha, Hunan 410127 (CN)
(74) Representative: Del Nero, Susanna
(86) International application number: PCT/CN2025/083516
(87) International publication number: WO 2026/060915

(57) **Abstract**

The present invention relates to a composition comprising a human milk oligosaccharide and a probiotic. The human milk oligosaccharide is a neutral fucosylated human milk oligosaccharide, and the probiotic is *Bifidobacterium animalis.* Specifically, the composition comprises 2'-fucosyllactose (2'-FL) and *Bifidobacterium animalis* CP-9. In addition, the present invention also relates to a use of the composition in the preparation of a food or a healthcare supplement, and a use of the composition in the preparation of a product for improving intestinal development.

## Description

### CROSS-REFERENCE TO THE RELATED APPLICATIONS

The present invention claims priority to Chinese Patent Application No. 2024113122316, filed with the China National Intellectual Property Administration on September 20, 2024 and entitled "COMPOSITION COMPRISING HUMAN MILK OLIGOSACCHARIDE AND PROBIOTIC, AND USE OF COMPOSITION IN IMPROVING INTESTINAL DEVELOPMENT", the entire contents of which are incorporated herein by reference.

### TECHNICAL FIELD

The present invention relates to a composition comprising a human milk oligosaccharide and a probiotic, and a use of the composition in the preparation of a product for improving development of a glycocalyx layer of intestinal epithelial cells. Specifically, the present invention relates to a composition of 2'-fucosyllactose (2'-FL) and *Bifidobacterium animalis* CP-9, and a use of the composition in the preparation of a product for improving development of a glycocalyx layer of intestinal epithelial cells.

### BACKGROUND

The intestinal barrier is considered to be the gatekeeper of human health. The intestinal barrier is responsible for the passage and absorption of nutrients, but also serves to prevent pathogens from entering the body. In addition, it regulates the interference between the intestinal lumen microbiota and macromolecules, and maintains tolerance and immune responses. After birth, the gatekeeping function of the neonatal intestinal barrier is not yet fully developed, and any disruption of the neonatal intestinal barrier function will have long-term effects and may play a role in the development of gastrointestinal infections and inflammatory bowel disease (IBD). In addition, intestinal barrier function also affects the development of other diseases in childhood and adulthood, such as obesity and allergy.

Human milk is the gold standard for infant nutrition, and human milk oligosaccharides (HMOs) are the main components that distinguish human milk from the milk of other mammals. In infant formula milk powder, an important function of HMOs is to stimulate the colonization of the microbiota in the gastrointestinal tract and to improve the development of intestinal barrier function. One possible way to achieve this is to stimulate the development of the epithelial glycocalyx on intestinal epithelial cells. The glycocalyx on neonatal intestinal epithelium provides binding sites for commensal microorganisms, and a well-developed glycocalyx can prevent the adhesion of pathogens and serve as a barrier to luminal toxins and enzymes, thereby promoting intestinal development. The ability of probiotics to adhere to the intestinal epithelium plays a key role in influencing intestinal flora composition, and colonization of strains on the intestinal epithelium competes with other species through greater adhesiveness. In recent years, many studies have shown that HMOs can enhance the adhesion ability of probiotics by affecting the expression of bacterial adhesins, but few studies have shown that HMOs can promote the adhesion ability of intestinal epithelial cells to probiotics by improving the development of the glycocalyx layer of intestinal epithelial cells.

Currently, there are few clinical intervention studies on the direct regulatory effects of HMOs on intestinal cells and structures. However, preclinical research data indicate that HMOs have a positive impact on intestinal maturation. *In vitro*, intestinal cell line cultures such as Caco-2 are commonly used to study the mechanisms by which nutrients and microbial metabolites affect epithelial integrity. It has been proven that Lacto-N-neotetraose (LnNT), 2'-fucosyllactose (2'-FL) and 6'-sialyllactose (6'-SL) can promote tight junction protein expression and increase cell differentiation along the crypt-villus axis; 2'-fucosyllactose and 3-fucosyllactose have positive effects on the expression of mucin glycoproteins. In *in vivo* animal experiments, it is found that supplementation with 2'-FL can significantly increase intestinal epithelial integrity and reduce the occurrence of colitis. Currently, there are few studies on the effects of a composition comprising a neutral fucosylated human milk oligosaccharide and *Bifidobacterium animalis* on the development of the glycocalyx layer of intestinal epithelial cells, and in particular, whether a synergistic promoting effect exists in the composition of the two on the development of the glycocalyx layer of intestinal epithelial cells is not yet clear.

Therefore, the present invention investigates the effect of a composition of 2'-FL and *Bifidobacterium animalis* CP-9 on glycocalyx development on intestinal epithelial cells at the cellular level, laying a theoretical foundation for the combined use of 2'-FL and *Bifidobacterium animalis* CP-9.

### SUMMARY

In a first aspect, an object of the present invention is to provide a composition comprising a human milk oligosaccharide and a probiotic, wherein the human milk oligosaccharide is a neutral fucosylated human milk oligosaccharide and the probiotic is *Bifidobacterium animalis*.

Specifically, the neutral fucosylated human milk oligosaccharide is 2'-fucosyllactose (2'-FL), and the *Bifidobacterium animalis* is *Bifidobacterium animalis* CP-9. The composition is an active ingredient of a food, a healthcare supplement or a medicament.

In an embodiment, the final concentration of the 2'-fucosyllactose (2'-FL) in the composition is 1.0 mg/mL to 2.6 mg/mL and/or the added concentration of the *Bifidobacterium animalis* is 10⁶ cfu/mL to 10⁸ cfu/mL.

For example, the final concentration of the 2'-fucosyllactose (2'-FL) is 1.0 mg/mL, 1.2 mg/mL, 1.4 mg/mL, 1.5 mg/mL, 1.6 mg/mL, 1.7 mg/mL, 1.8 mg/mL, 1.9 mg/mL, 2.0 mg/mL, 2.1 mg/mL, 2.2 mg/mL, 2.3 mg/mL, 2.4 mg/mL or 2.6 mg/mL.

In an embodiment, the concentration of the 2'-fucosyllactose (2'-FL) is 1.2 mg/mL to 2.4 mg/mL.

In a second aspect, another object of the present invention is to provide a use of a composition comprising a human milk oligosaccharide and a probiotic in the preparation of a food or a healthcare supplement.

In a third aspect, another object of the present invention is to provide a use of a composition comprising a human milk oligosaccharide and a probiotic in the preparation of a medicament for improving intestinal development.

In a fourth aspect, another object of the present invention is to provide a use of a composition comprising a human milk oligosaccharide and a probiotic in improving intestinal development.

In an embodiment, the above composition is configured to improve the development of the glycocalyx layer of intestinal epithelial cells. Specifically, the composition of 2'-fucosyllactose (2'-FL) and *Bifidobacterium animalis* CP-9 can synergistically improve the development of the glycocalyx layer on intestinal epithelial cells, thereby improving intestinal development.

In an embodiment, the manifestation of improving the development of the glycocalyx layer of intestinal epithelial cells is at least one selected from:

protecting a protein skeleton of glycocalyx, promoting hyaluronic acid synthesis of glycocalyx and promoting heparan sulfate synthesis of glycocalyx.

In an embodiment, the manifestation of improving the development of the glycocalyx layer of intestinal epithelial cells is at least one selected from: upregulating the transcription level of a glycocalyx development-related gene glypican 1, upregulating the transcription level of a gene encoding a hyaluronic acid synthase and upregulating the transcription level of a gene encoding an exostosin glycosyltransferase.

In an embodiment, the manifestation of improving the development of the glycocalyx layer of intestinal epithelial cells is at least one selected from: upregulating the transcription level of *gpc1*, the transcription level of *has1*, the transcription level of *has2*, the transcription level of *has3*, the transcription level of *ext1* and the transcription level of *ext2*.

In an embodiment, the composition has at least one of the following uses:
(1) 2'-fucosyllactose (2'-FL) improving the adhesion of *Bifidobacterium animalis* to intestinal epithelial cells; and
(2) *Bifidobacterium animalis* and 2'-FL synergistically promoting the development of the intestinal glycocalyx layer.

In an embodiment, 2'-fucosyllactose (2'-FL) improves the adhesion rate of intestinal epithelial cells to *Bifidobacterium animalis*.

In an embodiment, the composition is used to improve the transcription level of the encoding gene *gpc1* of Glypican 1 (GPC1) proteoglycan, thereby improving the protein skeleton development of the glycocalyx layer; improve the transcription levels of the encoding genes *has1*, *has2* and *has3* of hyaluronic acid synthase 1, thereby promoting the synthesis of hyaluronic acid synthase of the glycocalyx layer; and/or improve the transcription levels of the encoding genes *ext1* and *ext2* of exostosin glycosyltransferase 1, thereby promoting the synthesis of heparan sulfate of the glycocalyx layer.

In an embodiment, while 2'-fucosyllactose (2'-FL) improves the adhesion of *Bifidobacterium animalis* to intestinal epithelial cells, the *Bifidobacterium animalis* adhered to the intestinal epithelial cells further synergistically promotes the development of the intestinal glycocalyx layer with 2'-FL.

### BRIEF DESCRIPTION OF THE DRAWINGS

FIG. 1 shows the effect of 2'-FL on the adhesion rate of *Bifidobacterium animalis* CP-9 to Caco-2 cells.
FIG. 2 shows the effect of the composition of 2'-FL and *Bifidobacterium animalis* CP-9 on the *gpc1* transcription level of Caco-2 cells.
FIG. 3 shows the effect of the composition of 2'-FL and *Bifidobacterium animalis* CP-9 on the *has1* transcription level of Caco-2 cells.
FIG. 4 shows the effect of the composition of 2'-FL and *Bifidobacterium animalis* CP-9 on the *has2* transcription level of Caco-2 cells.
FIG. 5 shows the effect of the composition of 2'-FL and *Bifidobacterium animalis* CP-9 on the *has3* transcription level of Caco-2 cells.
FIG. 6 shows the effect of the composition of 2'-FL and *Bifidobacterium animalis* CP-9 on the *ext1* transcription level of Caco-2 cells.
FIG. 7 shows the effect of the composition of 2'-FL and *Bifidobacterium animalis* CP-9 on the *ext2* transcription level of Caco-2 cells.

### DETAILED DESCRIPTION OF THE EMBODIMENTS

In order that the objects, technical solutions and advantages of the embodiments of the present invention are clearer, the technical solutions of the present invention will be clearly and completely described below in combination with the following specific embodiments. Obviously, the specific embodiments described are part of the embodiments of the present invention. Based on the described embodiments of the present invention, all other embodiments obtained by those of ordinary skill in the art without creative effort shall fall within the scope of protection of the present invention.

The "glycocalyx" described in the present invention is composed of polysaccharides and proteins, and the adhesion of probiotics to host cells is also closely related to extracellular polysaccharides. Regulating the glycocalyx structure on the surface of intestinal epithelial cells or improving glycocalyx development may improve the mutual adhesion between probiotics and intestinal epithelial cells.

The "human milk oligosaccharide (HMO)" described in the present invention can be a neutral fucosylated human milk oligosaccharide, preferably 2'-fucosyllactose (2'-FL).

The glycocalyx is composed of polysaccharides and proteins. Proteoglycans are generally considered as the most important constituent components of the glycocalyx and form the skeleton of the glycocalyx. In addition, the glycocalyx contains glycosaminoglycan chains connected to the core proteins of proteoglycans, wherein heparan sulfate (HS) and hyaluronic acid (HA) are the main glycosaminoglycan components in the glycocalyx.

Therefore, in the present invention, the evaluation of the development of the glycocalyx layer of intestinal epithelial cells is mainly determined by measuring the transcription levels of the glycocalyx development-related genes glypican 1 (*gpc1*), hyaluronic acid synthase 1 (*has1*, *has2*, *has3*), and exostosin glycosyltransferase 1 (*ext1*, *ext2*) in Caco-2 cells. Specifically, *gpc1* is the encoding gene of Glypican 1 (GPC1) proteoglycan, and GPC1 is the protein skeleton of the glycocalyx layer and an important carrier of glycosaminoglycan chains including hyaluronic acid (HA) and heparan sulfate (HS). *has1*, *has2* and *has3* are encoding genes of hyaluronic acid synthase (HAS), HAS is essential for the synthesis of HA, and HA is a highly viscous component of the intestinal mucus layer and has functions of tissue repair, stability and anti-inflammatory action. Compared with HAS1 and HAS2, HAS3 synthesizes low molecular weight HA, and low molecular weight HA is essential for intestinal stem cell development. In addition, the *ext1* and *ext2* genes participate in the extension of heparan sulfate (HS) chains and are responsible for integrating HS chains with nucleotide sugars in the Golgi apparatus, and HS synthesis supports organogenesis, growth factor signaling and bacterial adhesion.

In a first aspect, an object of the present invention is to provide a composition comprising a human milk oligosaccharide and a probiotic, wherein the human milk oligosaccharide is a neutral fucosylated human milk oligosaccharide and the probiotic is *Bifidobacterium animalis*.

Specifically, the neutral fucosylated human milk oligosaccharide is 2'-fucosyllactose (2'-FL), and the *Bifidobacterium animalis* is *Bifidobacterium animalis* CP-9. The composition is an active ingredient of a food, a healthcare supplement or a medicament.

In an embodiment, the final concentration of the 2'-fucosyllactose (2'-FL) in the composition is 1.0 mg/mL to 2.6 mg/mL and/or the added concentration of the *Bifidobacterium animalis* is 10⁶ cfu/mL to 10⁸ cfu/mL.

For example, the final concentration of the 2'-fucosyllactose (2'-FL) is 1.0 mg/mL, 1.2 mg/mL, 1.4 mg/mL, 1.5 mg/mL, 1.6 mg/mL, 1.7 mg/mL, 1.8 mg/mL, 1.9 mg/mL, 2.0 mg/mL, 2.1 mg/mL, 2.2 mg/mL, 2.3 mg/mL, 2.4 mg/mL or 2.6 mg/mL.

In an embodiment, the concentration of the 2'-fucosyllactose (2'-FL) is 1.2 mg/mL to 2.4 mg/mL.

In a second aspect, another object of the present invention is to provide a use of a composition comprising a human milk oligosaccharide and a probiotic in the preparation of a food or a healthcare supplement.

In a third aspect, another object of the present invention is to provide a use of a composition comprising a human milk oligosaccharide and a probiotic in the preparation of a medicament for improving intestinal development.

In a fourth aspect, another object of the present invention is to provide a use of a composition comprising a human milk oligosaccharide and a probiotic in improving intestinal development.

In an embodiment, the above composition is used to improve the development of the glycocalyx layer of intestinal epithelial cells. Specifically, the composition of 2'-fucosyllactose (2'-FL) and *Bifidobacterium animalis* CP-9 can synergistically improve the development of the glycocalyx layer on intestinal epithelial cells, thereby improving intestinal development.

In an embodiment, the manifestation of improving the development of the glycocalyx layer of intestinal epithelial cells is at least one selected from:

protecting a protein skeleton of glycocalyx, promoting hyaluronic acid synthesis of glycocalyx and promoting heparan sulfate synthesis of glycocalyx.

In an embodiment, the manifestation of improving the development of the glycocalyx layer of intestinal epithelial cells is at least one selected from: upregulating the transcription level of a glycocalyx development-related gene glypican 1, upregulating the transcription level of a gene encoding a hyaluronic acid synthase and upregulating the transcription level of a gene encoding an exostosin glycosyltransferase.

In an embodiment, the manifestation of improving the development of the glycocalyx layer of intestinal epithelial cells is at least one selected from: upregulating the transcription level of *gpc1*, the transcription level of *has1*, the transcription level of *has2*, the transcription level of *has3*, the transcription level of *ext1* and the transcription level of *ext2*.

Within a certain concentration range, 2'-FL can significantly upregulate the transcription level of *gpc1*, and the addition of *Bifidobacterium animalis* CP-9 contributes to the upregulation of the transcription level of *gpc1*. When 2'-FL and *Bifidobacterium animalis* CP-9 are used in combination, by first adding 1.2 mg/mL to 2.4 mg/mL of 2'-FL to culture host cells, and then using *Bifidobacterium animalis* CP-9 adhered to the host cells for intervention, the relative transcription level of *gpc1* is significantly higher than that of using *Bifidobacterium animalis* CP-9 adhered to host cells alone for intervention. Therefore, 2'-FL and *Bifidobacterium animalis* CP-9 adhered to the host cells have a synergistic promoting effect on improving the relative transcription level of *gpc1*, and can protect the protein skeleton of glycocalyx.

Higher concentrations of 2'-FL and *Bifidobacterium animalis* CP-9 adhered to the host cells have synergistic promoting effects on improving the relative transcription levels of *has1*, *has2* and *has3*, and can promote hyaluronic acid synthesis of glycocalyx.

The combination of specific concentrations of 2'-FL and *Bifidobacterium animalis* CP-9 adhered to the host cells has a synergistic promoting effect on improving the transcription level of *ext1*, and can promote heparan sulfate synthesis of glycocalyx.

In an embodiment, the composition has at least one of the following uses:
(1) 2'-fucosyllactose (2'-FL) improving the adhesion of *Bifidobacterium animalis* to intestinal epithelial cells; and
(2) *Bifidobacterium animalis* and 2'-FL synergistically promoting the development of the intestinal glycocalyx layer.

In an embodiment, 2'-fucosyllactose (2'-FL) improves the adhesion rate of intestinal epithelial cells to *Bifidobacterium animalis*.

In an embodiment, the composition is used to improve the transcription level of the encoding gene *gpc1* of Glypican 1 (GPC1) proteoglycan, thereby improving the protein skeleton development of the glycocalyx layer; improve the transcription levels of the encoding genes *has1*, *has2* and *has3* of hyaluronic acid synthase 1, thereby promoting the synthesis of hyaluronic acid synthase of the glycocalyx layer; and/or improve the transcription levels of the encoding genes *ext1* and *ext2* of exostosin glycosyltransferase 1, thereby promoting the synthesis of heparan sulfate of the glycocalyx layer.

In an embodiment, while 2'-fucosyllactose (2'-FL) improves the adhesion of *Bifidobacterium animalis* to intestinal epithelial cells, the *Bifidobacterium animalis* adhered to the intestinal epithelial cells further synergistically promotes the development of the intestinal glycocalyx layer with 2'-FL.

### Examples

The examples of the present invention are only used to exemplarily describe the technical solutions of the present invention, but do not mean that the technical solutions of the present invention are limited to these specific examples.

The inventors of the present application have proved through specific experiments that the composition of 2'-fucosyllactose (2'-FL) and *Bifidobacterium animalis* CP-9 of the present invention has a synergistic promoting effect in improving the development of the glycocalyx layer of intestinal epithelial cells, and that 2'-fucosyllactose (2'-FL) improves the adhesion of *Bifidobacterium animalis* to intestinal epithelial cells.

The experimental methods and test substances used in each example and control are as follows.

The culture methods for Caco-2 cells and *Bifidobacterium animalis* (CP-9) used in the examples are as follows.

### 1. Culture of Caco-2 Cells

Human colon adenocarcinoma cells (Caco-2, purchased from Nanjing Senbeijia Biological Technology Co., Ltd.) are cultured in MEM complete medium containing 20% premium fetal bovine serum (purchased from Nanjing Senbeijia Biological Technology Co., Ltd.), grown in 25 cm² cell culture flasks, and placed in a carbon dioxide incubator at 37°C, 5% CO₂ and 95% relative humidity for culture. The culture medium is changed every one or two days. When cells grew to 80% to 90% confluence, 0.25% trypsin digestion solution is used for digestion, and subculture is performed at a ratio of 1:3.

### 2. Culture of Bifidobacterium animalis (CP-9)

The lyophilized preserved *Bifidobacterium animalis* CP-9 (purchased from Anhui JinQiao Biological Technology Co., Ltd.) is inoculated into modified MRS medium (purchased from Hope Biological Technology Co., Ltd.). After culture at 37°C for 24 h, it is inoculated into fresh modified MRS medium (purchased from Hope Biological Technology Co., Ltd.) at 1%. After culture at 37°C for 24 h, the pour plate method is used for viable bacterial counting, and the viable bacterial count of *Bifidobacterium animalis* CP-9 after the second activation is recorded.

### Example 1

This example provides an experiment on the effect of 2'-FL on the adhesion of *Bifidobacterium animalis* CP-9.
(1) The Caco-2 cell density was adjusted to 1×10⁵ cells/cm², and inoculated in a 24-well plate, and 2'-FL at a final concentration of 0.6 mg/mL was added to the MEM medium for co-incubation for 3 days. The cultured *Bifidobacterium animalis* CP-9 was centrifuged to remove the supernatant, washed twice with sterile PBS, and resuspended in MEM complete medium, and the CP-9 viable bacterial concentration was adjusted to 1×10⁸ CFU/mL bacterial suspension.
(2) The supernatant of the incubated Caco-2 cell culture medium was removed, and MEM complete medium (without double antibiotics) containing 2'-FL at an added concentration of 0.6 mg/mL and *Bifidobacterium animalis* CP-9 at an added concentration of 1×10⁸ CFU/mL was added, and incubation was continued in a 37°C, 5% CO₂ incubator for 2 h. The liquid in the wells was discarded, and then gently washed twice with sterile PBS solution to remove unadhered bacteria. 150 µL trypsin cell digestion solution was added, and after the cells completely detached, 350 µL complete cell culture medium was added to terminate digestion, and 10-fold gradient dilution was performed.

The number of adhered bacteria was determined by the pour plate colony counting method, the adhesion rate was calculated according to formula (1), and Blank Group 1 without added 2'-FL was used as a control.$adhension rate \left(%\right) = \frac{adhered viable bacterial count}{initial viable bacterial count} \times 100$

### Example 2

This example provides an experiment on the effect of 2'-FL on the adhesion of *Bifidobacterium animalis* CP-9. (1) The final concentration of 2'-FL in MEM was 1.2 mg/mL; and (2) the supernatant of the incubated Caco-2 cell culture medium was removed, and MEM complete medium (without double antibiotics) containing 2'-FL at an added concentration of 1.2 mg/mL and *Bifidobacterium animalis* CP-9 at an added concentration of 1×10⁸ CFU/mL was added, and incubation was continued in a 37°C, 5% CO₂ incubator for 2 h. Other steps are the same as in Example 1.

### Example 3

This example provides an experiment on the effect of 2'-FL on the adhesion of *Bifidobacterium animalis* CP-9. (1) The final concentration of 2'-FL in MEM was 2.4 mg/mL; and (2) the supernatant of the incubated Caco-2 cell culture medium was removed, and MEM complete medium (without double antibiotics) containing 2'-FL at an added concentration of 2.4 mg/mL and *Bifidobacterium animalis* CP-9 at an added concentration of 1×10⁸ CFU/mL was added, and incubation was continued in a 37°C, 5% CO₂ incubator for 2 h. Other steps are the same as in Example 1.

The specific concentrations of 2'-FL and CP-9 strain in Blank Group 1 and Examples 1-3 are shown in Table 1 below.

**Table 1**

| | 2'-FL Content in MEM | CP-9 Strain Concentration |
|---|---|---|
| Blank Group 1 | 0 mg/mL | 1×10⁸ CFU/mL |
| Example 1 | 0.6 mg/mL | 1×10⁸ CFU/mL |
| Example 2 | 1.2 mg/mL | 1×10⁸ CFU/mL |
| Example 3 | 2.4 mg/mL | 1×10⁸ CFU/mL |

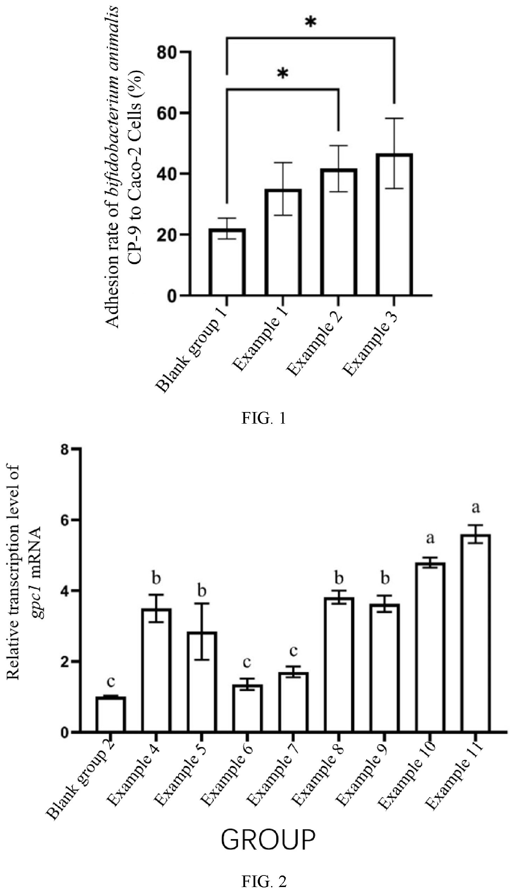

The results are shown in FIG. 1. Compared with Blank Group 1, after Caco-2 cells are co-cultured with different concentrations of 2'-FL, the adhesion rate of Caco-2 cells to *Bifidobacterium animalis* CP-9 is increased. In particular, under the intervention of 1.2 mg/mL and 2.4 mg/mL of 2'-FL, the adhesion rate of Caco-2 cells to *Bifidobacterium animalis* CP-9 is significantly increased compared with the adhesion rate of Blank Group 1, increasing by 1.89 times and 2.12 times, respectively (p < 0.05).

The following examples test the effects of 2'-FL and CP-9 on glycocalyx development of intestinal epithelial cells.

### Examples 4 to 5

The Caco-2 cell density was adjusted to 1×10⁵ cells/cm², and inoculated in a 24-well plate, and a specific concentration of 2'-FL was added to the MEM complete medium and co-incubated with Caco-2 cells for 48 h, wherein 1.2 mg/mL of 2'-FL was added in Example 4, and 2.4 mg/mL of 2'-FL was added in Example 5. After incubation, total cellular RNA was extracted, and reverse transcription and qPCR determination were performed.

### Examples 6 to 7

The Caco-2 cell density was adjusted to 1×10⁵ cells/cm², and inoculated in a 24-well plate, and cells were cultured with MEM complete medium for 36 h. When the confluence exceeded 70%, a specific number of viable bacteria of *Bifidobacterium animalis* CP-9 was added, wherein 10⁷ cfu/mL of *Bifidobacterium animalis* CP-9 was added in Example 6, and 10⁸ cfu/mL of *Bifidobacterium animalis* CP-9 was added in Example 7. After 2 h of incubation, washing was performed twice with sterile PBS solution, and only *Bifidobacterium animalis* CP-9 adhered to Caco-2 cells was retained. Referring to the method in Section 2.1, the adhesion of *Bifidobacterium animalis* CP-9 to Caco-2 cells in 3 replicate wells was determined (see Table 2 below), and the remaining 3 replicate wells were supplemented with MEM complete medium and incubated for another 10 h, then total RNA was extracted, and reverse transcription and qPCR determination were performed.

### Examples 8 to 11

The Caco-2 cell density was adjusted to 1×10⁵ cells/cm², and inoculated in a 24-well plate, and MEM complete medium containing a specific concentration of 2'-FL was added for co-incubation for 36 h. When the confluence exceeded 70%, a specific number of viable bacteria of *Bifidobacterium animalis* CP-9 was added, and after 2 h of incubation, washing was performed twice with sterile PBS solution, and only *Bifidobacterium animalis* CP-9 adhered to Caco-2 cells was retained. Referring to the method in Example 1, the adhesion of *Bifidobacterium animalis* CP-9 to Caco-2 cells in 3 replicate wells was determined (see Table 2 below), and the remaining 3 replicate wells were supplemented with MEM complete medium containing a final concentration of 1.2 mg/mL of 2'-FL, and incubated for another 10 h, then total RNA was extracted, and reverse transcription and qPCR determination were performed.

Specifically, in Example 8, MEM complete medium containing 1.2 mg/mL of 2'-FL was used for co-incubation for 36 h, and when the confluence exceeded 70%, 10⁷ cfu/mL of *Bifidobacterium animalis* CP-9 was added, and the measured adhesion concentration of *Bifidobacterium animalis* CP-9 to Caco-2 cells was 10⁶ cfu/mL; in Example 9, MEM complete medium containing 2.4 mg/mL of 2'-FL was used for co-incubation for 36 h, and when the confluence exceeded 70%, 10⁷ cfu/mL of *Bifidobacterium animalis* CP-9 was added, and the measured adhesion concentration of *Bifidobacterium animalis* CP-9 to Caco-2 cells was 10⁶ cfu/mL; in Example 10, MEM complete medium containing 1.2 mg/mL of 2'-FL was used for co-incubation for 36 h, and when the confluence exceeded 70%, 10⁸ cfu/mL of *Bifidobacterium animalis* CP-9 was added, and the measured adhesion concentration of *Bifidobacterium animalis* CP-9 to Caco-2 cells was 10⁷ cfu/mL; and in Example 11, MEM complete medium containing 2.4 mg/mL of 2'-FL was used for co-incubation for 36 h, and when the confluence exceeded 70%, 10⁸ cfu/mL of *Bifidobacterium animalis* CP-9 was added, and the measured adhesion concentration of *Bifidobacterium animalis* CP-9 to Caco-2 cells was 10⁷ cfu/mL.

The specific concentrations of the added substances and the intervention substances 2'-FL and CP-9 in each example are shown in Table 2 below. At the same time, MEM medium is set as Blank Group 2 as a control.

qPCR was performed using the qPCR primer sequences shown in Table 3 below to determine the transcription levels of the glycocalyx development-related genes glypican 1 (*gpc1*), hyaluronic acid synthase 1 (*has1*, *has2*, *has3*), and exostosin glycosyltransferase 1 (*ext1*, *ext2*) in Caco-2 cells.

**Table 2**

| Group Name | Added Substance | Intervention Substance |
|---|---|---|
| Blank Group 2 | - | - |
| Example 4 | 1.2 mg/mL 2'-FL | 1.2 mg/mL 2'-FL |
| Example 5 | 2.4 mg/mL 2'-FL | 2.4 mg/mL 2'-FL |
| Example 6 | 10⁷ cfu/mL CP-9 | 10⁶ cfu/mL CP-9 (adhesion concentration) |
| Example 7 | 10⁸ cfu/mL CP-9 | 10⁷ cfu/mL CP-9 (adhesion concentration) |
| Example 8 | 1.2 mg/mL 2'-FL + 10⁷ cfu/mL CP-9 | 1.2 mg/mL 2'-FL + 10⁶ cfu/mL CP-9 (adhesion concentration) |
| Example 9 | 1.2 mg/mL 2'-FL + 10⁸ cfu/mL CP-9 | 1.2 mg/mL 2'-FL + 10⁷ cfu/mL CP-9 (adhesion concentration) |
| Example 10 | 2.4 mg/mL 2'-FL + 10⁷ cfu/mL CP-9 | 2.4 mg/mL 2'-FL + 10⁶ cfu/mL CP-9 (adhesion concentration) |
| Example 11 | 2.4 mg/mL 2'-FL + 10⁸ cfu/mL CP-9 | 2.4 mg/mL 2'-FL + 10⁷ cfu/mL CP-9 (adhesion concentration) |

**Table 3**

| Name | Forward Primer | SEQ ID NO |
|---|---|---|
| *gpc1* | 5'-TATTGCCGAAATGTGCTCAAGGGC | 1 |
| *has1* | 5'-CCACCCAGTACAGCGTCAAC | 2 |
| *has2* | 5'-TCTTTATGTGACTCATCTGTCTCACCGG | 3 |
| *has3* | 5'-TATACCGCGCGCTCCAA | 4 |
| *ext1* | 5'-CATAGGCGATGAGAGATTGT | 5 |
| *ext2* | 5'-GGCTACGATGTCAGCATTCCTG | 6 |

| Name | Reverse Primer | SEQ ID NO |
|---|---|---|
| *gpc1* | 5'-ATGACACTCTCCACACCCGATGTA | 7 |
| *has1* | 5'-CATGGTGCTTCTGTCGCTCT | 8 |
| *has2* | 5'-TTGTTGGCTACCAGTTTATCCAAACG | 9 |
| *has3* | 5'-GCCACTCCCGGAAGTAAGACT | 10 |
| *ext1* | 5'-CAAGAATTGTGTCTGCTGTC | 11 |
| *ext2* | 5'-GGCTTCTAGGTCCTCTCTGTAC | 12 |

### (I) Evaluation of the Effects of 2'-FL and CP-9 in Different Examples on the Protein Skeleton of Glycocalyx Layer

According to the above experimental preparation steps and specific experimental methods, the results of the relative transcription levels of the obtained *gpc1* mRNA are shown in FIG. 2.

It can be seen that different concentrations of 2'-FL in Examples 4 to 5 can significantly upregulate the relative transcription level of *gpc1* (*p* < 0.05), but as the concentration of 2'-FL increases, the relative transcription level of *gpc1* shows a downward trend.

According to the *gpc1* mRNA relative transcription levels of Examples 6 to 7, it can be found that when only *Bifidobacterium animalis* CP-9 adhered to Caco-2 is retained as the intervention substance, the relative transcription level of *gpc1* of Caco-2 shows no significant change compared with Blank Group 2 (*p* > 0.05), but as the adhered strain increases, the *gpc1* relative transcription level shows an upward trend.

According to the relative transcription levels of *gpc1* mRNA of Examples 8 to 11, it can be known that after first adding 1.2 mg/mL to 2.4 mg/mL of 2'-FL to culture Caco-2 cells, and then using *Bifidobacterium animalis* CP-9 adhered to Caco-2 for intervention, the relative transcription level of *gpc1* is significantly higher than that of using only *Bifidobacterium animalis* CP-9 adhered to Caco-2 for intervention (Examples 6 to 7) (*p* < 0.05). At the same time, as the concentration of 2'-FL increases, the relative transcription level of *gpc1* shows an upward trend (*p* < 0.05), and when 2.4 mg/mL of 2'-FL is first added, that is, in Examples 10 to 11, the relative transcription levels of *gpc1* are significantly higher than those of the other examples (*p* < 0.05).

In summary, 2'-FL and *Bifidobacterium animalis* CP-9 adhered to Caco-2 cells have a synergistic promoting effect on improving the relative transcription level of *gpc1*, and can protect the protein skeleton of glycocalyx.

### (II) Evaluation of the Effects of 2'-FL and CP-9 in Different Examples on Hyaluronic Acid Synthase of Glycocalyx Layer

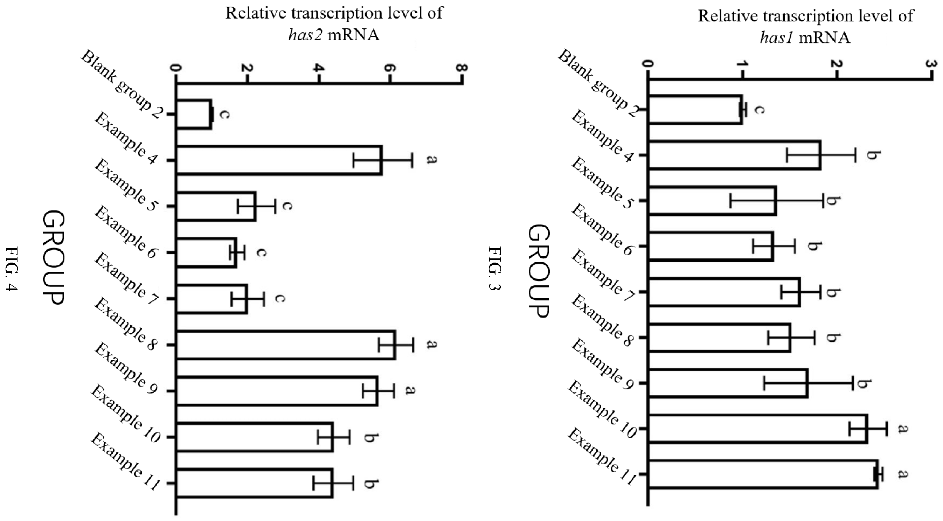

According to the above experimental preparation steps and specific experimental methods, the results of the relative transcription levels of the obtained *has1*, *has2* and *has3* mRNA are shown in FIGS. 3 to 5.

Different concentrations of 2'-FL in Examples 4 to 5 can significantly upregulate the transcription level of *has1* (*p* < 0.05), and 1.2 mg/mL of 2'-FL can significantly upregulate the relative transcription levels of *has2* and *has3*, and as the concentration of 2'-FL increases, the relative transcription levels of *has1*, *has2* and *has3* all show a downward trend.

According to Examples 6 to 7, it can be found that when only adhered *Bifidobacterium animalis* CP-9 is retained as the intervention substance, only the relative transcription level of *has1* in Caco-2 can be significantly upregulated, and the degree of improvement of the mRNA relative transcription levels of *has2* and *has3* in Caco-2 is small.

According to the relative transcription levels of *has1*, *has2* and *has3* mRNA of Examples 8 to 11, it can be found that after first adding 1.2 mg/mL to 2.4 mg/mL of 2'-FL to culture Caco-2 cells, and then using *Bifidobacterium animalis* CP-9 adhered to Caco-2 for intervention, the mRNA relative transcription levels of *has1* are all significantly higher than the mRNA relative transcription levels of *has1* in Examples 4 to 7 (*p* < 0.05). At the same time, the mRNA relative transcription levels of *has2* and *has3* in Examples 10 and 11 are significantly higher than those in Examples 5 to 7 (using 2'-FL at the added concentration of 2.4 mg/mL alone or using CP-9 at the adhered concentration of 10⁶ cfu/mL to 10⁷ cfu/mL alone as the intervention substance) (*p* < 0.05).

In summary, higher concentrations of 2'-FL and *Bifidobacterium animalis* CP-9 adhered to Caco-2 cells have synergistic promoting effects on improving the relative transcription levels of *has1*, *has2* and *has3*, and can promote hyaluronic acid synthesis of glycocalyx.

### (III) Evaluation of the Effects of 2'-FL and CP-9 in Different Examples on Heparan Sulfate of Glycocalyx Layer

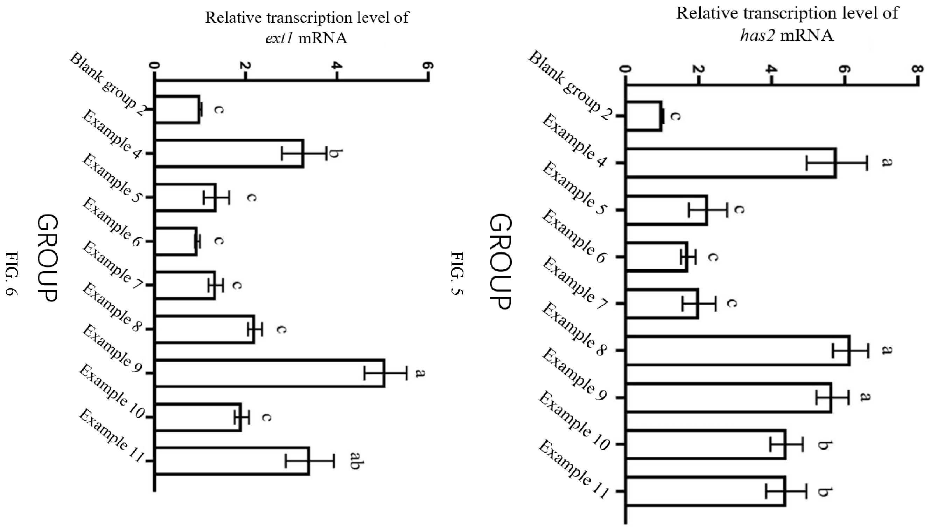

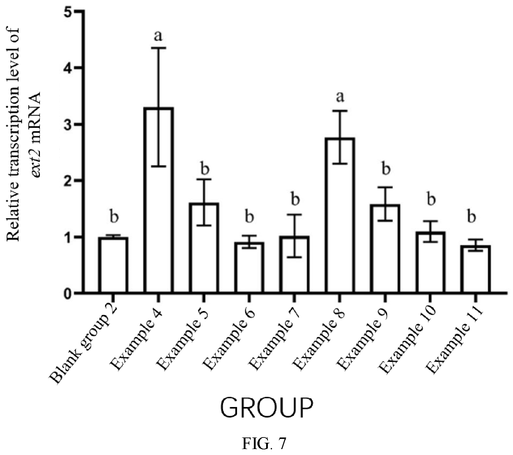

According to the above experimental preparation steps and specific experimental methods, the results of the relative transcription levels of the obtained *ext1* and *ext2* mRNA are shown in FIGS. 6 to 7.

It can be seen that among different concentrations of 2'-FL in Examples 4 to 5, only 1.2 mg/mL of 2'-FL can significantly upregulate the transcription levels of *ext1* and *ext2*, and as the concentration of 2'-FL increases, the transcription levels of *ext1* and *ext2* show a downward trend.

According to the mRNA relative transcription levels of *ext1* and *ext2* in Examples 6 to 7, it can be found that when only *Bifidobacterium animalis* CP-9 adhered to Caco-2 is retained as the intervention substance, the relative transcription levels of *ext1* and *ext2* in Caco-2 show no significant difference compared with Blank Group 2 (*p* > 0.05).

According to the mRNA relative transcription levels of *ext1* in Examples 8 to 11, it can be found that after first adding 1.2 mg/mL to 2.4 mg/mL of 2'-FL to culture Caco-2 cells, and then using *Bifidobacterium animalis* CP-9 adhered to Caco-2 for intervention, the *ext1* transcription levels in Examples 8 to 9 are significantly higher than those of the other examples (*p* < 0.05).

According to the mRNA relative transcription levels of *ext2* in Examples 8 to 11, it can be found that after first adding 1.2 mg/mL to 2.4 mg/mL of 2'-FL to culture Caco-2 cells, and then using *Bifidobacterium animalis* CP-9 adhered to Caco-2 for intervention, the *ext2* transcription levels show no significant improvement compared with the *ext2* transcription levels of Examples 4 to 7, but rather decreases compared with the *ext2* transcription levels of using different concentrations of 2'-FL alone.

According to Examples 6 to 7, it can be known that when CP-9 is used alone as the intervention substance, CP-9 has no effect on promoting the gene transcription levels of *ext1* and *ext2* in Caco-2, which may be because the strain itself or its metabolites have no upregulating effect on or have a certain inhibitory effect on the gene transcription levels of *ext1* and *ext2*. Therefore, after first adding 2'-FL to promote the adhesion of *Bifidobacterium animalis* CP-9, the effect of *Bifidobacterium animalis* CP-9 on the gene transcription levels of *ext1* and *ext2* (no upregulation or inhibitory effect) is amplified, and therefore no synergistic promoting effect on the *ext2* transcription level is exhibited.

It is confirmed that 2'-FL and *Bifidobacterium animalis* CP-9 adhered to Caco-2 cells need to be used at specific concentrations to exhibit a synergistic promoting effect on the gene transcription levels of *ext1* and *ext2*, and it is not a simple superposition of effects.

In summary, the combination of specific concentrations of 2'-FL and *Bifidobacterium animalis* CP-9 adhered to Caco-2 cells has a synergistic promoting effect on improving the transcription level of *ext1*, and can promote heparan sulfate synthesis of glycocalyx.

### INDUSTRIAL APPLICABILITY

Using a composition containing 2'-fucosyllactose (2'-FL) and *Bifidobacterium animalis* CP-9 in a specific concentration range can synergistically improve the development of the glycocalyx layer of intestinal epithelial cells, prevent the adhesion of pathogens through good development of the glycocalyx layer of intestinal epithelial cells, provide good binding sites for commensal microorganisms, and further promote the development of intestinal barrier function and intestinal development.

Using 2'-fucosyllactose (2'-FL) in a specific concentration range can improve the adhesion rate of intestinal epithelial cells to *Bifidobacterium animalis*, thereby influencing the composition of intestinal flora and promoting the development of intestinal barrier function and intestinal development.

## Claims

1. A composition, comprising a human milk oligosaccharide and a probiotic, **characterized in that** the human milk oligosaccharide is a neutral fucosylated human milk oligosaccharide and the probiotic is *Bifidobacterium animalis*.

2. The composition according to claim 1, wherein the neutral fucosylated human milk oligosaccharide is 2'-fucosyllactose (2'-FL).

3. The composition according to claim 1, wherein the *Bifidobacterium animalis* is *Bifidobacterium animalis* CP-9.

4. The composition according to any one of claims 1 to 3, wherein a final concentration of the 2'-fucosyllactose (2'-FL) in the composition is 1.0 mg/mL to 2.6 mg/mL and/or an added concentration of the *Bifidobacterium animalis* is 10⁶ cfu/mL to 10⁸ cfu/mL.

5. The composition according to any one of claims 1 to 3, wherein a concentration of the 2'-fucosyllactose (2'-FL) is 1.2 mg/mL to 2.4 mg/mL.

6. A use of the composition according to any one of claims 1 to 5 in a preparation of a food or a healthcare supplement.

7. A use of the composition according to any one of claims 1 to 5 in a preparation of a medicament for improving intestinal development.

8. A use of the composition according to any one of claims 1 to 5 in improving intestinal development.

9. The use according to claim 7 or 8, wherein the composition improves development of a glycocalyx layer of intestinal epithelial cells.

10. The use according to claim 9, wherein a manifestation of improving the development of the glycocalyx layer of intestinal epithelial cells is at least one selected from:
protecting a protein skeleton of glycocalyx, promoting hyaluronic acid synthesis of glycocalyx and promoting heparan sulfate synthesis of glycocalyx.

11. The use according to claim 10, wherein the manifestation of improving the development of the glycocalyx layer of intestinal epithelial cells is at least one selected from: upregulating transcription level of a glycocalyx development-related gene glypican 1, upregulating transcription level of a gene encoding a hyaluronic acid synthase and upregulating transcription level of a gene encoding an exostosin glycosyltransferase.

12. The use according to claim 11, wherein the manifestation of improving the development of the glycocalyx layer of intestinal epithelial cells is at least one selected from: upregulating transcription level of *gpc1*, transcription level of *has1*, transcription level of *has2*, transcription level of *has3*, transcription level of *ext1* and transcription level of *ext2*.

13. The use according to any one of claims 7 to 12, wherein the composition has at least one of following uses:
(1) 2'-fucosyllactose (2'-FL) improving adhesion of the *Bifidobacterium animalis* to the intestinal epithelial cells; and
(2) the *Bifidobacterium animalis* and 2'-FL synergistically promoting development of an intestinal glycocalyx layer.
